# EUROPÄISCHE PATENTANMELDUNG

(11) **EP 0 560 099 A2**
(43) Veröffentlichungstag der Anmeldung: **15.09.1993**
(21) Anmeldenummer: 93102638.9
(22) Anmeldetag: 19.02.1993
(51) Int. Cl.: B01L 3/00, A61B 5/20, G01N 33/52

(54) **Vorrichtung zur Erfassung und/oder Messung oder Kontrolle der Beschaffenheit, insbesondere chemischer und/oder biologischer Verhältnisse mit Hilfe von wenigstens einem Indikator in flüssigem Milieu, insbesondere wässrigem Milieu, wie z.B. Urin**

(30) Priorität: 26.02.1992 DE 4205894
(71) Anmelder: RAHE, Martin, D-32609 Hüllhorst (DE)
(72) Erfinder: RAHE, Martin, D-32609 Hüllhorst (DE)

(57) **Zusammenfassung**

1. Vorrichtung zur Erfassung und/oder Messung oder Kontrolle der Beschaffenheit, insbesondere chemischer Verhältnisse mit Hilfe von wenigstens einem Indikator in flüssigem Milieu, insbesondere wässrigen Milieu.
2. Harninkontinenz beinhaltet ein Höchstmaß an Infektionsrisiko.
2.1 Die bisher verwandten Indikatorteststreifen erfordern eine Inkorbation der Teststreifen im Urin für eine bestimmte Zeit und eine anschließende Auswertung. Dies führt dazu, daß die Meßmethode nicht oder unzureichend häufig durchgeführt wird.
Aufgabe der Erfindung ist, eine Vorrichtung zu schaffen, die sich durch eine genaue Meßmöglichkeit bei einfachster Handhabung auszeichnet.
2.2 Diese Aufgabe wird dadurch gelöst, daß die Wandung der Vorrichtung wenigstens eine das flüssige Milieu über einen vorbestimmten zu definierenden Zeitraum in das Vorrichtungsinnere einlassenden Durchlaß aufweist, daß eine das flüssige Milieu in eine Bewegung in das Vorrichtungsinnere veranlassende erste Einrichtung vorgesehen ist und eine den in das Vorrichtungsinnere stattfindenden Flüssigkeitseinlauf stoppende zweite Einrichtung vorgesehen ist.
2.3 Die Vorrichtung kann zur Früherkennung bei Infektionen bei Harninkontinenz zum Einsatz gelangen.

## Beschreibung

Die Erfindung betrifft eine Vorrichtung zur Erfassung und/oder Messung oder Kontrolle der Beschaffenheit, insbesondere chemischer und/oder biologischer Verhältnisse mit Hilfe von wenigstens einem Indikator in flüssigem Milieu, insbesondere wässrigem Milieu, wie z.B. Urin mit den Merkmalen des Oberbegriffes des Patentanspruches 1.

Harninkontinenz bzw. Harnverhaltung ist bei traumatischen Rückenmarksschädigungen (z.B. Querschnittslähmung) und ähnlichen Veränderungen das zentralen Nervensystem oder als Begleiterscheinung einer Vielzahl anderer Krankheiten ein sehr verbreitetes Problem. Neben den persönlichen Unannehmlichkeiten für die Betroffenen beinhaltet sie ein Höchstmaß an Infektionsrisiko.

Solche Infektionen der ableitenden Harnwege und harnverarbeitenden Organe stellen nicht nur durch Dauermedikamentation und lange Krankenhausaufenthalte einen erheblichen Kostenfaktor dar; sie sind auch darüber hinaus die häufigste Todesursache bei dieser Personengruppe.

Die Erkennung bzw. Kontrolle einer Infektion ist die einzige Möglichkeit durch frühzeitig gezielten medikamentösen Einsatz die Risiken zu reduzieren.

Dieses Problem soll durch bereits am Markt vorhandene Indikator-Teststreifen gelöst werden, mit denen eine Infektion frühzeitig festgestellt werden kann. Derartige Teststreifen setzen aber eine umständliche Inkubation der Teststreifen im Urin für eine bestimmte Zeit und eine dann folgende direkte Auswertung voraus.

In der Praxis wird deshalb diese Meßmethode nicht oder nur mit unzureichender Häufigkeit durchgeführt, da derartige Tests bei in aller Regel unsachgemäßer Behandlung bzw. Durchführung allenfalls zu ungenauen Ergebnissen führen und aus diesem Grund eher schädlich als nutzbringend sind.

Desweiteren wird versucht, dieses Problem durch eine scheckkartengroße Indikatorkarte zu lösen, die z.B. auf die Innnenseite einer durchsichtigen Kunststoff-Folie eines Einweg-Urinals oder einer vergleichbaren Urinsammeleinrichtung aufgebracht werden kann. Der Aufbau der Indikatorkarte umfaßt dabei einen Indikatorbereich, der z.B. Nitrit, Leukozyten und PH-Werte durch Farbumschlag anzeigt und nachfolgend, mit Hilfe einer spektralen Zuordnungsskale, die Auswertung des Harnes gestattet.

Aber auch diese Lösung führt zu keinem befriedigenden Ergebnis, da auch nicht sichergestellt ist, daß stets eine gleichbleibende Urinmenge an den Indikatorträger gelangt, so daß sowohl aufgrund der verschiedenen Urinmengen und damit Sättigungsgrade des Indikators als auch der Gefahr von Auswaschungen die Ergebnisse nicht befriedigend sind.

Aufgabe der Erfindung ist es daher, eine Vorrichtung zur Erfassung und/oder Messung der Beschaffenheit von flüssigen Milieu, insbesondere wässrigem Milieu wie z.B. Urin zu schaffen, die ohne manuelles hinzu tun sicherstellt, daß grade so viel Körperflüssigkeitsmenge an den Indikator gelangt, wie für die spezifische Umsetzung mit dem Indikatorfarbstoff erforderlich ist und dies bei gleichzeitig einfachster und damit wirksamster Handhabung.

Diese Aufgabe wird bei einer Vorrichtung der eingangs genannten Gattung mit den Merkmalen des kennzeichnenden Teiles des Patentanspruches 1 gelöst. Weitere vorteilhafte Ausgestaltungen der Erfindung sind im Gegenstand der Unteransprüche.

Dadurch, daß die Wandung wenigstens einen, das flüssige Milieu über einen vorbestimmten zu definierenden Zeitraum in das Vorrichtungsinnere einlassenden Durchlaß aufweist, daß eine das flüssige Milieu in eine Bewegung in das Vorrichtungsinnere veranlassende erste Einrichtung vorgesehen ist und eine den in das Vorrichtungsinnere stattfindenden Flüssigkeitseinlauf stoppende zweite Einrichtung vorgesehen ist, wird sichergestellt, daß zum einen die Einlaßmenge schon durch die Größe der Öffnung gesteuert werden kann und zum anderen die Flüssigkeit über eine aktive Einrichtung zu einem Einfließen in die Vorrichtung veranlaßt wird und durch die zweite Einrichtung der Flüssigkeitseinlauf gestoppt und nicht nur wie bisher üblich verlangsamt wird, so daß Testergebnisse erzielt werden können, die sich durch eine hohe Genauigkeit auszeichnen, da stets die gleiche zu messende Flüssigkeitsmenge herangeführt wird und dies nicht in Abhängigkeit von der jeweiligen Genauigkeit des Bedieners der Vorrichtung. Desweiteren wird durch die Möglichkeit der Farbkonservierung in der erfindungsgemäßen Vorrichtung sichergestellt, daß auch nach Ablauf einer relativ langen Zeit noch mit ausreichend hoher Genauigkeit des Meßergebnis abgelesen werden kann.

Bildet der die Vorrichtung aufnehmende Träger im Bereich der Vorrichtung eine den Vorrichtungsinnenraum begrenzende Wand und wird der Innenraum gemeinsam mit der den Vorrichtungsinnenraum wenigstens teilweise umhüllenden Wendung definiert, so ist dadurch die Möglichkeit geschaffen, die Vorrichtung z.B. auf eine sogenannte Check-up-Karte, bekannt aus der DE 39 31 659 aufzubringen, wobei zumindest der Abschnitt der Checkkarte im Bereich der erfindungsgemäßen Vorrichtung dann durchsichtig ausgestaltet sein muß, so daß ohne das eine derartige Checkkarte jeweils gehändelt werden müßte, das Meßergebnis abgelesen werden kann und dadurch die Möglichkeit besteht, eine derartige Checkkarte z.B. in einer Auffangvorrichtung für unkontrolierbar ausgeschiedenen Urin anzubringen oder aber auch in z.B. Windeln, um jeweils die entsprechenden Ergebnisse ablesen zu können.

Ist die Wandung wenigstens teilweise diffusionsdicht ausgestaltet, wird erreicht, daß auch über größere Zeiträume hinweg z.B. ein Unterdruck in der Vorrichtung gehalten werden kann.

Ist die Wandung an ihrer Außenseite wenigstens teilweise mit einem im flüssigen Milieu, insbesondere wässrigem Milieu, wie z.B. Urin, sich auflösenden Film, insbesondere durch Überzug abgedichtet ausgestaltet, so wird dadurch sichergestellt, daß vor einer Messung bzw. Einbringung der Vorrichtung in das zu messende Milieu zu keinerlei Fremdeinwirkungen auf das Innere der Vorrichtung kommt, so daß das Innere der Vorrichtung steril und damit mit der notwendigen Genauigkeit arbeiten kann, das heißt unter "Laborbedingungen".

Ist die den Vorrichtungsinnenraum wenigstens teilweise umhüllende Wandung eine Membran und ist der in der Membran angeordnete Durchlaß als Perforation ausgestaltet, so ist dadurch zum einen eine einfache und wirkungsvolle Form der Öffnung gewählt worden und zum andern sichergestellt, daß etwaige in der zu messenden Flüssigkeit vorhandenen Verunreinigungen nicht in das Vorrichtungsinnere gelangen können, so daß schon beim Eindringen der Flüssigkeit für eine erste Vorfilterung und damit Meßgenauigkeit gesorgt wird.

Ist zwischen dem den Durchlaß beinhaltenden Teil der Membran und dem Indikator eine Flüssigkeits- oder Milieusteuerungseinrichtung vorgesehen, so ist dadurch sichergestellt, daß das zu messende Milieu zunächst einmal eine gewisse Distanz zwischen der Membran und dem Indikator zurückzulegen hat und so ein weiterer Filtereffekt auftritt, so daß lediglich das zu messende Substrat an den Indikator gelangt und nicht etwaig vorhandene Verunreinigungen und zum weiteren ein Stützelement vorliegt, das der Vorrichtung den notwendigen Halt gibt.

Ist die Flüssigkeits- oder Milieusteuerungseinrichtung als eine mit Mikrokapseln und mit Kapillaren versehene die Einrichtung zur Veranlassung der Bewegung des flüssigen Milieus in das Vorrichtungsinnere darstellenden Quellsubstanz ausgebildet, sind diese Kapselschalen semipermeable wasserdurchlässig ausgestaltet und ist die zweite den Flüssigkeitseinlauf stoppende Einrichtung ebenfalls die mikroverkapselte eine semipermeable Kapselschale aufweisende und durch Aufnahme von Flüssigkeit ihr Volumen vergrößernde Quellsubstanz, so wird dadurch erreicht, daß bei Aufnahme von Wasser durch die Volumenvergrößerung beim Quellvorgang die Kapillare zugedrückt und damit die Durchlässigkeit aufgehoben wird. Die Größe und Anzahl der Kapillare bestimmt dadurch sowohl die Quellreaktion als auch die Durchlaßzeit bzw. die Durchlaßmenge des zu messenden Milieus.

Desweiteren wird durch die semipermeable Kapselschale erreicht, daß es nicht zu etwaigen Diffusionen durch die Quellsubstanz kommen kann, so daß sichergestellt ist, daß nach dem Schließen der Kapilaren kein weiteres, z.B. wässriges Milieu nachfließen kann.

Dieser Effekt kann noch weiter erhöht werden, in dem zwischen der Flüssigkeits- und/oder Milieusteuerungseinrichtung und dem Indikator eine mit Durchlässen zu den Kapillaren versehene Sperrschicht angeordnet wird.

Durch die Ausgestaltung der Flüssigkeits- und/oder Milieusteuerungseinrichtung bzw. durch die Bereitstellung der Flüssigkeits- und/oder Milieusteuerungseinrichtung aus einem mit einem wasserlöslichen Hüllmaterial mikroverkapselten und mit Kapillaren versehenen, die Einrichtung zur Veranlassung der Bewegung des flüssigen Milieus in das Vorrichtungsinnere darstellende wasserundurchlässiges Dichtungsmittel und dadurch, daß diese Flüssigkeits- und/oder Milieusteuerungseinrichtung ebenfalls die zweite den Flüssigkeitseinlauf stoppende Einrichtung darstellt, in dem nach Auflösung derwasserlöslichen Kapselschale das Dichtungsmittel freigesetzt wird und die Kapillare schließt, wird ebenfalls eine Flüssigkeits- und/oder Milieusteuerungseinrichtung bereitgestellt, die sich zum einen durch hohe Betriebseicherheit und zum anderen durch geringe Produktionskosten auszeichnet.

Besteht die Flüssigkeits- und/oder Milieusteuerungseinrichtung, wie auch in einem weiteren Ausführungsbeispiel, aus einem hydrophoben Material, das mit Kapillaren versehen ist und dessen Oberflächen mit einer wasserlöslichen hydrophilen Substanz versehen sind, so wird dadurch erreicht, daß zum einen eine ganz bestimmte Wasser- bzw. Milieumenge durch die Kapillaren fließen kann, gleichzeitig aber nach Auflösen der hydrophilen Substanz es zu einer Unterbrechung des kapillaren flusses kommt, wobei die transportierte, z.B. Urinmenge, in einem bestimmten Verhältnis zu der Löslichkeit und der Menge der hydrophilen Substanz steht, so daß auch hier die Harnmenge genau zu steuern ist.

Durch die Ausgestaltung der den Vorrichtungsinnenraum wenigstens teilweise umhüllenden Wandung als Folie und die Ausgestaltung des Durchlasses als einer in der Folie angeordnete Perforation, wobei die Öffnung wenigstens an ihrer Außenseite durch eine wasserlösliche oder chemisch/physisch reaktive Substanz geschlossen ist und die Folie eine diffusionsdichte metallbedampfte Folie ist, wird erreicht, daß innerhalb der Vorrichtung, hier insbesondere im Bereich des Indikatorträgers ein Unterdruck herrscht, so daß die einzufließende Milieumenge über den Unterdruck geregelt werden kann, bzw. genau festgelegt werden kann, ohne daß jedoch die Gefahr besteht, daß bei z.B. einer längeren Lagerung der Unterdruck durch die Membran entweicht.

Gleichzeitig ist ebenfalls sichergestellt, daß in der Vorrichtung sterile Verhältnisse herrschen, da der Durchlaß verschlossen ist. Erst bei Einsatz der Vorrichtung in dem zu messenden Milieu, löst sich der Verschluß des Durchlasses auf und es kann, hervorgerufen durch den Unterdruck, Flüssigkeit in die Vorrichtung gelangen, wobei nach Abbau des Unterdrucks dann der entsprechende Fluß zum Stillstand kommt.

Weist der Indikatorträger im Bereich der Öffnung eine wenigstens teilkugelförmige Einbuchtung auf, so ist sichergestellt, daß nach Abbau des Unterdruckes es nicht zu etwaigen kapillaren Wirkungen, das heißt zu einem kapilaren Fluß kommen kann, da die Flüssigkeits- und/oder Milieusteuerungseinrichtung nicht unmittelbar mit dem vor der Vorrichtung anstehenden Flüssigkeiten in Kontakt steht.

Ist wenigstens der den Durchlaß aufweisende Teil der den Innenraum der Vorrichtung wenigstens teilweise definierenden und umhüllenden Wandung elastisch ausgestaltet und ist der elastisch ausgestaltete Membranteil hervorgerufen durch den im Vorrichtungsinneren herrschenden Unterdruck in einer vorgespannten nach innen gerichteten Position angeordnet, so kommt es nach dem lösen des die Membran umhüllenden Schutzfilmes und nach lösen des den Durchlaß verschließenden Verschlusses zu einem Eindringen von zu messender Flüssigkeit, so daß sich die Membran entspannen kann und aus ihrer vorgespannten Position in eine entspannte Position verfährt, wodurch es zu einer Volumenvergrößerung des Vorrichtungsinnenraumes kommt und dadurch zu einem mechanisch hervorgerufenen gewissen Unterdruck, der ebenfalls eine Sogwirkung auf, vor der Vorrichtung befindliche Flüssigkeit ausübt, so daß es zu einem Flüssigkeitsfluß in das Vorrichtungsinnere, hervor gerufen einmal durch das vorher vorhandene Vakuum und zum zweiten durch die Entspannung der Membran kommt.

Ist im Bereich des den Durchlaß aufweisenden Teiles der Membran eine perforierte Folie angeordnet und ist die perforierte Folie derart angeordnet, daß zwischen ihr und der unter Vorspannung stehenden Membran ein Hohlraum entsteht, so ermöglicht dies die Nutzung dieses Hohlraumes z.B. zur Anbringung eines sogenannten Luftkissens, wenn z.B. ein wenigstens die perforierte Folie abdichtender wasserlöslicher Schutzfilm vorgesehen ist, so daß die elastische Membran zum einen wie bisher, durch einen in der Vorrichtung herrschenden Unterdruck in die vorgespannte Position verbracht werden kann, oder aber zum anderen durch das anzuordnende Luftpolster und als dritte Möglichkeit sowohl durch das Luftpolster als auch gleichzeitig durch den Unterdruck, so daß beide, das heißt sowohl das Luftpolster als auch der Unterdruck nur in einem recht geringen Umfang vorhanden sein müssen.

Weist die perforierte Folie eine sogenannte Dichtungsfläche auf, die dem in der perforierten Membran angeordneten Durchlaß gegenüberliegend angeordnet ist, so bietet dies die Möglichkeit, daß nach Entspannen der Membran oder ein aktives Vorbringen der Membran durch z.B. einen Quellvorgang der Flüssigkeits- und/oder Milieusteuerungseinrichtung die Durchlaßöffnung gegen die Dichtungsfläche im Dichteschluß anliegt, so daß der Flüssigkeitseinlauf zum erliegen kommt.

Desweiteren ermöglicht die Schaffung eines Hohlraumes zwischen Folie und flexibler bzw. elastisch ausgestattete Membran die Positionierung einer sogenannten Stütz- oder Fixiersubstanz, um die Membran in eine entsprechende Ausgangsposition zu verbringen.

Ist diese Stütz- oder Fixiersubstanz mit einer Kapillare aufweisenden und ihr Volumen vergrößernden Substanz, insbesondere Kunststoff versetzt, so hat dies zur Folge, daß bei Eindringen von entsprechender Flüssigkeit es zum einen zu einer Volumenvergrößerung kommt und zum anderen dadurch zu einer sogenannten Sprengwirkung, das heißt die Stütz- oder Fixiersubstanz wird auf schnellstmöglichem Wege zerlegt, so daß es zu einer Entspannung der elastisch vorgespannten Membran kommen kann und dadurch zu einem Ansaugen von einer genau definierten Flüssigkeitsmenge in den Indikatorbereich.

Ist die Flüssigkeits- und/oder Milieusteuerungseinrichtung durch Kontakt mit flüssigen Milieu volumenvergrößernd ausgestaltet, so wird dadurch die Möglichkeit geschaffen, den elastischen Teil der Membran gegen z.B. eine Dichtungsfläche zu drücken, um sodann einen Flüssigkeitsfluß nachhaltig zu stoppen. Dabei kann die Membran im Bereich der Dichtungsfläche mit einem Kleber versehen sein, so daß es bei einer entsprechenden Berührung zu einem Verkleben von Membran und Dichtungsfläche kommt.

In der Zeichnung sind 16 Ausführungsbeispiele der erfindungsgemäßen Vorrichtung zur Erfassung bzw. Messung oder Kontrolle der Beschaffenheit, insbesondere chemischer und/oder biologischer Verhältnisse mit Hilfe von wenigstens einem Indikator, schematisch dargestellt, und zwar zeigt
Fig. 1 den Aufbau einer mit der erfindungsgemäßen Vorrichtung versehenen "Check-up-Karte" im Querschnitt,
Fig. 1a rückseitige Ansicht in Blickrichtung Ader mit der Vorrichtung versehenen Check-up-Karte,
Fig. 2 die erfindungsgemäße Vorrichtung gemäß Ausführungsbeispiel 1 im Querschnitt,
Fig. 3 die erfindungsgemäße Vorrichtung gemäß Ausführungsbeispiel 2 im Querschnitt,
Fig. 4 die erfindungsgemäße Vorrichtung gemäß Ausführungsbeispiel 3 im Querschnitt,
Fig. 5 die erfindungsgemäße Vorrichtung gemäß Ausführungsbeispiel 4 im Querschnitt,
Fig. 6a bis Fig. 6d die erfindungsgemäße Vorrichtung gemäß der Ausführungsbeispiele 5 bis 8 im Querschnitt,
Fig. 7 die erfindungsgemäße Vorrichtung gemäß Ausführungsbeispiel 9 im Querschnitt,
Fig. 8 die erfindungsgemäße Vorrichtung gemäß Ausführungsbeispiel 10 im Querschnitt,
Fig. 9 die erfindungsgemäße Vorrichtung gemäß Ausführungsbeispiel 11 im Querschnitt,
Fig. 10 die erfindungsgemäße Vorrichtung gemäß Ausführungsbeispiel 12 im Querschnitt,
Fig. 10a die erfindungsgemäße Vorrichtung gemäßAusführungsbeispiel 12 im Querschnitt ohne Perforation,
Fig. 11 die erfindungsgemäße Vorrichtung gemäß Ausführungsbeispiel 13 im Querschnitt,
Fig. 12 die erfindungsgemäße Vorrichtung gemäß Ausführungsbeispiel 14 im Querschnitt und
Fig. 13 die erfindungsgemäße Vorrichtung gemäß Ausführungsbeispiel 15 im Querschnitt.

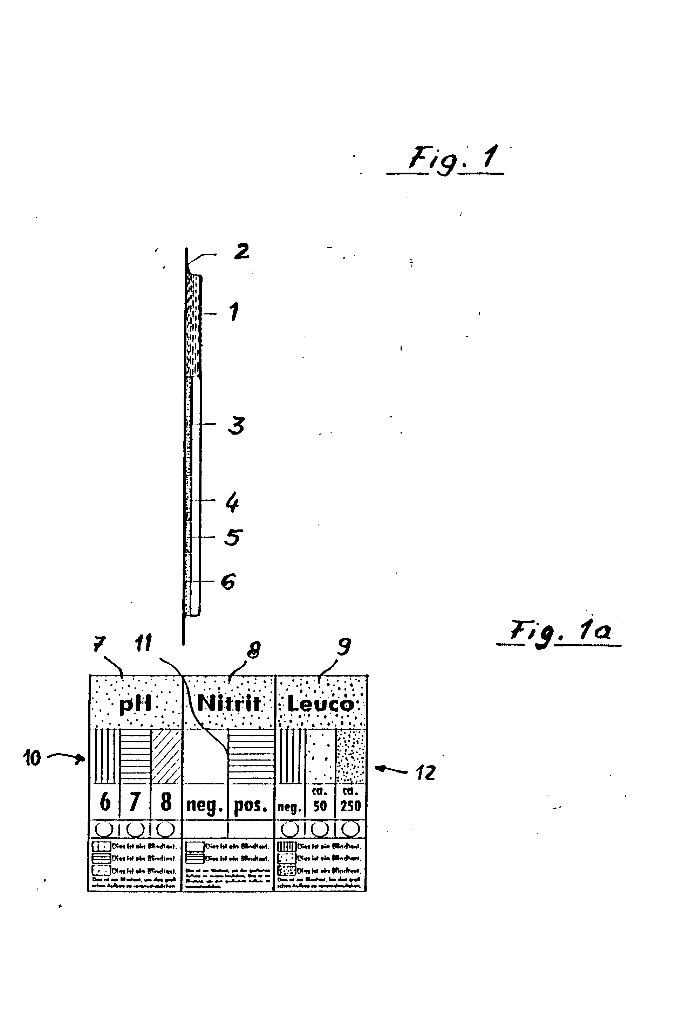

Wie aus Fig. 1 ersichtlich, kann die erfindungsgemäße Vorrichtung (1) vorteilhafterweise bei einer Check-up-Karte (2), bekannt aus der DE 39 31 659 zum Einsatz gelangen, wobei die Check-up-Karte (2) gemäß Fig. 1 an ihrem in Blickrichtung Fig. 1 oberen Ende die erfindungsgemäße Vorrichtung (1) zur Erfassung bzw. Messung der Beschaffenheit, insbesondere chemischer Verhältnisse in flüssigem Milieu, insbesondere wässrigem Milieu, wie z.B. Urin aufweist.

Unter der erfindungsgemäßen Vorrichtung (1) kann sich dann, wie im Ausführungsbeispiel 1 dargestellt, eine Farben-Vergleichsskala (3) und daran anschließend ein Bestimmungsfeld (4) sowie ein Beschriftungsfeld (5) und eine Informationsbeschriftung (6) anschließen, wobei die mit der erfindungsgemäßen Vorrichtung (1) versehene Check-up-Karte (2) dann auf die Innenseite einer hier nicht dargestellten durchsichtigen Kunststoff-Folie eines Einweg-Urinals oder einer vergleichbaren Urinsammeleinrichtung aufgebracht werden kann.

Es ist jedoch ebenso denkbar, daß eine derartige Check-up-Karte (2) z.B. in Kinderwindeln oder vergleichbaren Inkontinenzsystemen bei Erwachsenen angebracht werden können.

Die erfindungsgemäße Vorrichtung (1) kann dabei, dargestellt in Fig. 1a, z.B. drei getrennt voneinander angebrachte Bereiche (7;8;9) aufweisen, sogenannte Indikatorenabschnitte (7,8,9), wobei jedem dieser Indikatorenabschnitte (7,8,9) jeweils eine entsprechende Vergleichsfarbenkarte (10,11,12) zugeordnet ist, ebenso wie die entsprechende Bestimmungsfelder (4), Beschriftungsfelder (5) und eine entsprechende Informationsbeschriftung (6).

Die Vorrichtungen (1) zur Erfassung bzw. Messung der Beschaffenheit, insbesondere chemischer und/oder biologischer Verhältnisse mit Hilfe von wenigstens einem Farbindikator im wässrigen Milieu können dabei einen verschiedenartigen Aufbau aufweisen.

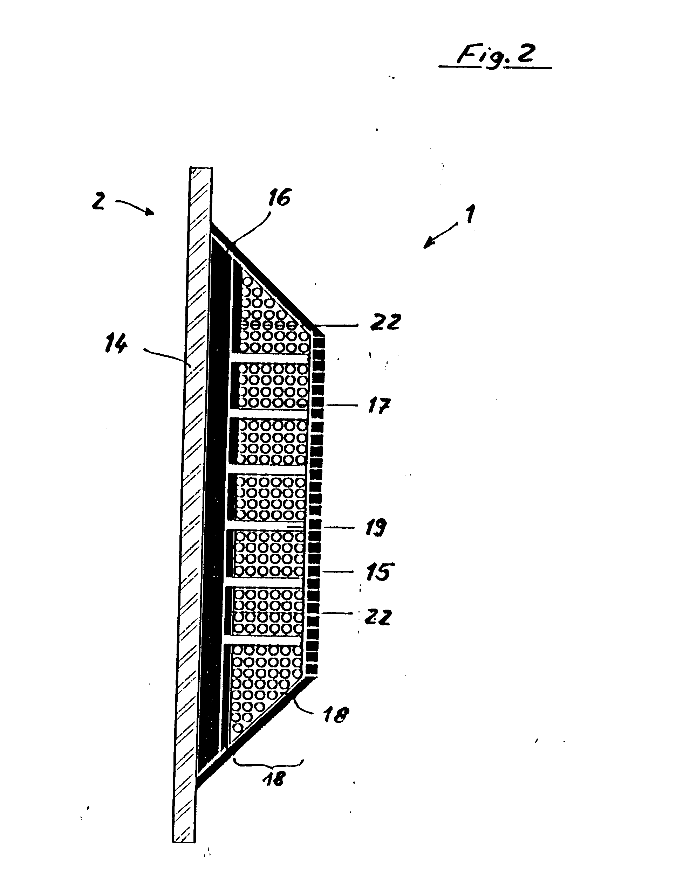

Wie aus Fig. 2 ersichtlich, ist die erfindungsgemäße Vorrichtung (1) derart auf der Check-up-Karte (2) aufgebracht, daß diese, ausgerichtet zur z.B. urinexpornierten Seite, das heißt in Blickrichtung Fig. 2 rechts von der Karte (2) eine Membran (13) aufweist, die gemeinsam mit dem, eine Wand (14) der Vorrichtung (1) bildenden Teil der Check-up-Karte eine trapezförmige Ausgestaltung, wobei der parallal zur Karte (2) bzw. zum die Rückwand (14) bildenden Teil der Karte (2) verlaufende Teil (15) der Membran (13) perforiert ausgestaltet ist.

Im Inneren der Vorrichtung (1) ist unmitelbar an der Rückwand (14) angrenzend der Indikator (16) derart angeordnet, daß er sich parallel zur Rückwandinnenseite erstreckend befindet.

Dieser Indikator (16) ist in den hier vorliegenden Ausführungsbeispielen als Farbindikator ausgebildet.

Zwischen dem Indikator (16) und der Membran (13) bzw. dem perforierten Membranabschnitt (15) befindet sich eine mikroverkapselte Quellsubstanz (17), die gleichzeitig als Flüssigkeits- und Milieustauerungseinrichtung und Stützbereich (18) fungiert und mit Durchgangsöffnungen bzw. Kapillare (19) versehen ist, wobei diese Durchgangsöffnungen (19) im hier vorliegenden Ausführungsbeispiel kanalförmig als Kapillare ausgestaltet sind.

Bei Aufnahme von Wasser oder einem sonstigen wässrigen Milieu wird durch die mikroverkappselte Quellsubstanz (17) verhindert, daß es zu einer Defusion des z.B. Urins über die Quellsubstanz (17) an den Indikator (16) kommt, da die semipermeable Kapselschale der mikroverkapselten Quellsubstanz (17) die Flüssigkeit in der Kapsel bindet und es nach Aufnahme einer entsprechenden Flüssigkeitsmenge zu einer Volumenvergrößerung, das heißt zu einem Quellvorgang, kommt, der die Durchgangslöcher (19) bzw. Kapillare (19) zudrückt und damit die Durchlässigkeit der mikroverkapselten Quellsubstanzschicht (17) aufhebt.

Die Größe und Anzahl der Löcher in der Membran (15) sowie die Größe und Anzahl der Durchgangskapilare (19) in der Quellsubstanz (17) bestimmt neben der Quellreaktion die Durchlaßzeit und Durchlaßmenge, so daß über die entsprechende Ausgestaltung der erfindungsgemäßen Vorrichtung (1) sowohl die Durchlaßzeit als auch die Durchlaßmenge des zu messenden Milieus exakt zu steuern ist.

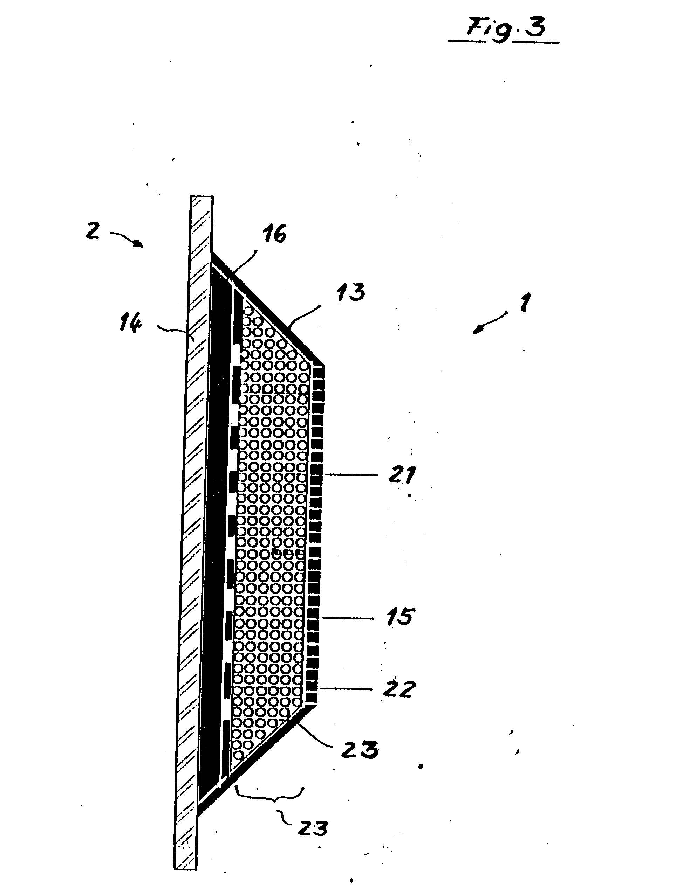

Gemäß Fig. 3 ist die erfindungsgemäße Vorrichtung (1) auch hier auf einen Träger (14) in diesem Ausführungsbeispiel ebenfalls auf die Check-up-Karte (2) angeordnet, so daß der Träger gleichzeitig als Rückwand (14) der erfindungsgemäßen Vorrichtung (1) fungiert.

In direktem Kontakt zu dem Träger, das heißt zu der Rückwand (14), befindet sich ebenfalls auch hier der Indikator (16), wobei die äußere Begrenzung der erfindungsgemäßen Vorrichtung (1) auch in diesem Ausführungsbeispiel neben der Trägerkarte bzw. Rückwand (14) durch eine sogenannte Membran (13) gebildet wird, die ebenfalls in Blickrichtung Fig. 3 in Zusammenspiel mit der Rückwand (14), das heißt der Karte (2) eine trapezförmige Ausgestaltung aufweist und deren parallel zur Karte (2) verlaufende Seite (15) mit einer entsprechenden die zu messende Flüssigkeit einlassende Perforierung ausgestattet ist.

Zwischen der Membran (13) bzw. dem die Perforierung aufweisenden Teil (15) und dem Indikator (16) ist hier im vorliegenden Ausführungsbeispiel ein mikroverkapseltes wasserunlösliches Dichtungsmittel (21) angeordnet, das mit entsprechenden Kapilaren versehen ist, so daß die zu messende Flüssigkeit durch diese Schicht hindurchtreten kann.

Zwischen der aus einem mikroverkapselten Dichtungsmittel (21) bestehenden Schicht und dem Indikator (16) ist eine sogenannte Sperrschicht (22) platziert, mit konstruktiv eingegrenzten Durchgangsbereichen.

Bei Durchtritt eines wässrigen Milieus, wie z.B. Urin, durch die Perforation der Membran (15), löst sich die aus einem wasserlöslichen Hüllmaterial bestehende Mikroverkapselung auf, so daß das wasserunlösliche Dichtungsmittel freigesetzt wird und den Flüssigkeits- und/oder Milieusteuerungsbereich bzw. die Einrichtung (23) abdichten kann, so daß keine Flüssigkeit bzw. keine weitere Flüssigkeit zum Indikator (16) gelangen kann.

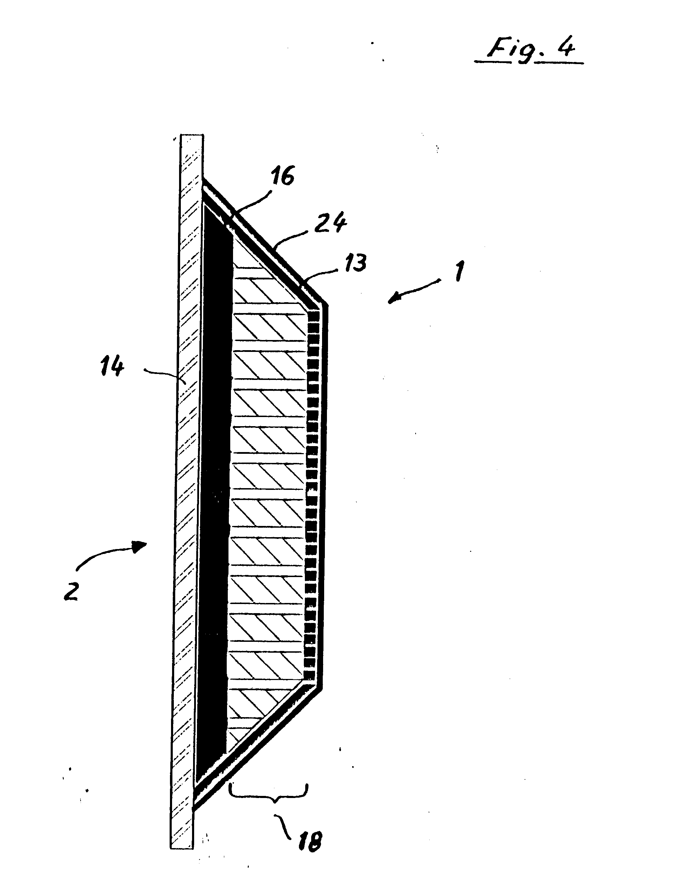

Im Ausführungsbeispiel 3 gemäß Fig. 4 ist die erfindungsgemäße Vorrichtung (1) ebenfalls auf einer Trägerkarte (2) angeordnet, so daß das Innere der erfindungsgemäßen Vorrichtung (1) auch hier durch die Trägerkarte (2) und eine darauf trapezförmig ausgestaltete Membran (13) umhüllt wird.

Unmittelbar an die Trägerkarte (2) bzw. der Rückwand (14) angrenzend ist auch in diesem Ausführungsbeispiel der Indikator (16) angeordnet, wobei zwischen der der urinexponierten Seite zugewandten Seite des Indikators (16), das heißt der in Blickrichtung Fig. 4 rechten Seite des Indikators (16) und der Membraninnenseite ebenfalls ein Flüssigkeits- und/oder Milieusteuerungseinrichtung bzw. -bereich (18), der im hier vorliegenden Ausführungsbeispiel aus einem hydrophoben Material besteht, das von einer wasserlöslichen hydrofilen Substanz umgeben ist, so daß die Kapilaren zu Beginn einer etwaigen Aufnahme eines wässrigen Milieus bzw. des Urins zunächst einmal in direkten Kontakt mit der hydrophilen Substanz stehen, angeordnet.

Durch diese hydrophile Substanz werden die Kapillare bzw. die Kapillareigenschaften derart bestimmt, daß das System zunächst Wasserleitfähig ist. Beim Benetzen der durchlässigen Schutzmembran mit z.B. Urin, das heißt beim Benetzen der hydrophilen Substanz bzw. Kapillarbeschichtung wid diese gelöst, so daß bis zur Auflösung der hydrophilen Schicht eine geringe definierte Urinmenge in Richtung der Indikatorsubstanz transportiert wird.

Nach Auflösung dieser Schicht, sind die Kapillare nun durch das unmittelbare Anstehen der hydrophoben Substanz nicht nur für eine kapillare Leitung des wässrigen Milieus geeignet.

Durch den daraus resultierenden kapillaren Verschluß kann kein weiterer Harn nachgeleitet werden.

Die transportierte Urinmenge steht in einem vorbestimmten bzw. genau definierten Verhältnis zu der Löslichkeit und der Menge der hydrophilen Substanz, die die Kapillaritätseigenschaften verändert.

Da der Indikatorbereich, das heißt das Innere der Vorrichtung (1) steril bleiben muß und soll, können die Systemöffnungen, das heißt die Perforation der Membran (13) mit einem wasser und/oder harnlöslichen Film (24) abgedichtet werden, der bei einer Benetzung mit dem zu messenden bzw. zu erfassenden wässrigen Milieu wie z.B. Urin, nach einer vorbestimmten Zeit aufgelöst wird, so daß es erst nach Benetzen der erfindungsgemäßen Vorrichtung (1) mit dem zu messenden z.B. Urin zu einer Öffnung der Perforation kommt und dadurch sichergestellt ist, daß die erfindungsgemäße Vorrichtung (1) bis zu einer entsprechenden Messung des wässrigen Milieus steril bleibt.

Dieser lösliche Film kann sich bis in die Perforationen hinein erstrecken, so daß es bei einer Auflösung zu einem ersten Fluss der Flüssigkeit kommt.

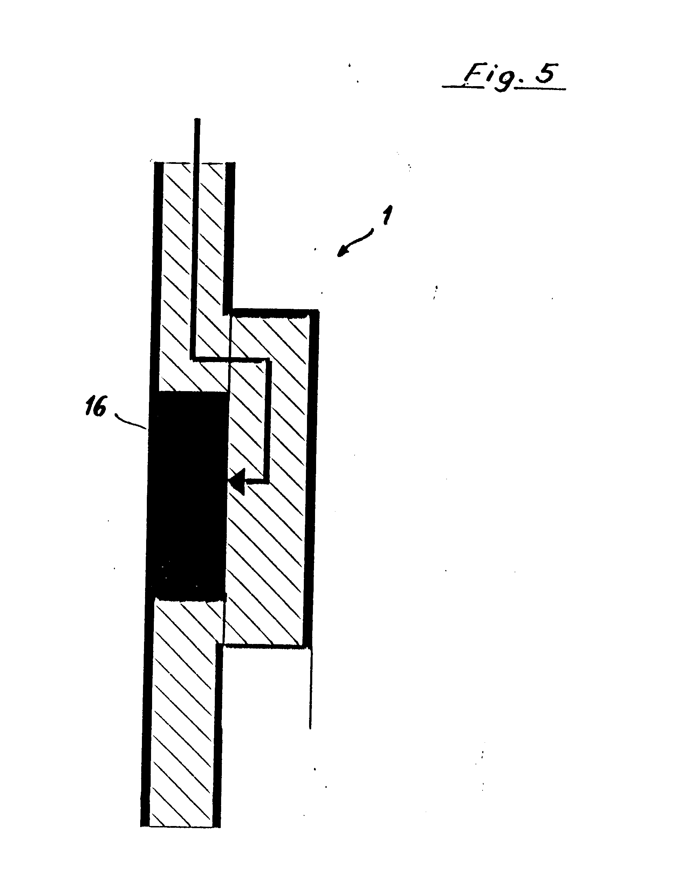

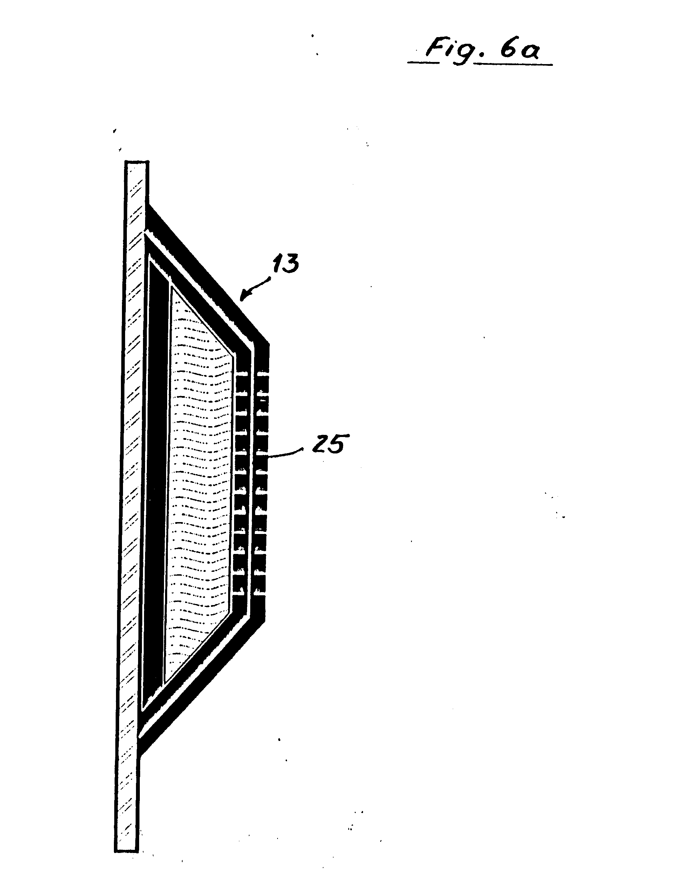

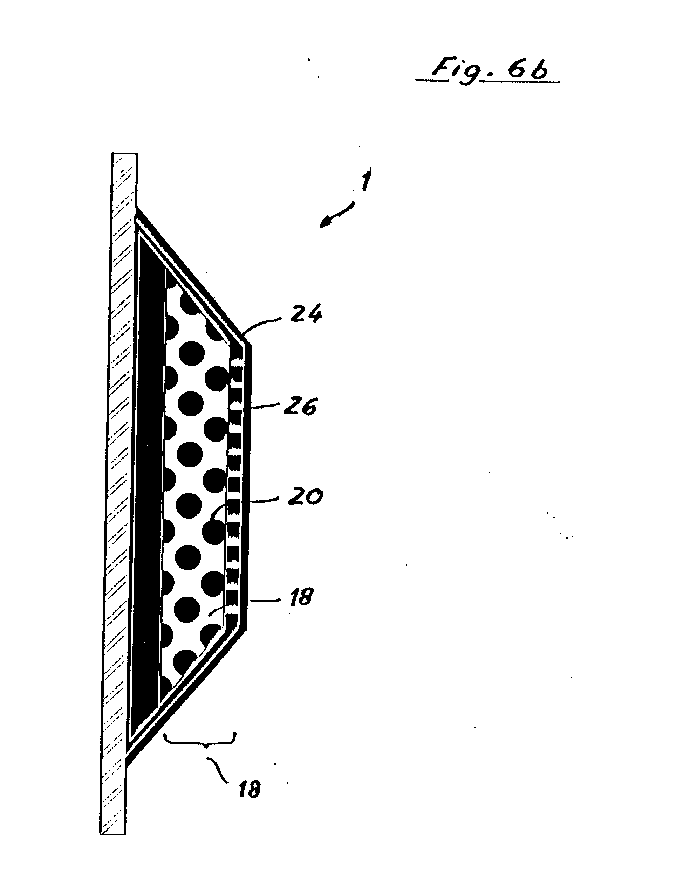

Bei dem in Fig. 5 dargestellten Ausführungsbeispiel handelt es sich weitgehend um den gleichen Aufbau wie zuvor geschildert, jedoch wird hier das zu messende Milieu nicht durch eine der Indikatorfläche gegenüberliegende perforierte Membran (15) geleitet, sondern vielmehr in Blickrichtung Fig. 5 über eine seitlich bzw. ober- und unterhalb des Indikators (16) angeordnete Öffnung, so daß der kapillare Transportweg beliebig verlängert werden kann, ohne daß dies zu einer störenden Vergrößerung der Breite bzw. Tiefe der erfindungsgemäßen Vorrichtung (1) führt.

Die zu messende wässrige Lösung wird dabei ebenfalls durch einen kapillaren Bereich geführt, der durch eine hydrophile Substanz gebildet wird, die nach der Benetzung in Auflösung übergeht und den hydrophoben Kernbereich freigibt, so daß es auch hier zu einer Beendigung der Flüssigkeitszufuhr kommt und dadurch zu einer genau vorher zu bestimmenden Messdauer und Heranführung einer genau definierten Flüssigkeitsmenge.

Dieser Konstruktionsaufbau gleichen Prinzips ermöglicht eine beliebige Verlängerung des kapillaren Transportweges, ohne die Systemdicke erheblich vergrößern zu müssen. Hier wird prinzipiell wie bei der vorherigen Version, der Urin über den Rand des Systems transportiert und gelangt in den hydrophilen Bereich hinter die Indikatorsubstanz. Dieser Bereich ermöglicht die gleichmäßige Verteilung des Urins. Außerdem können dort Überschüsse kapillar festgehalten werden.

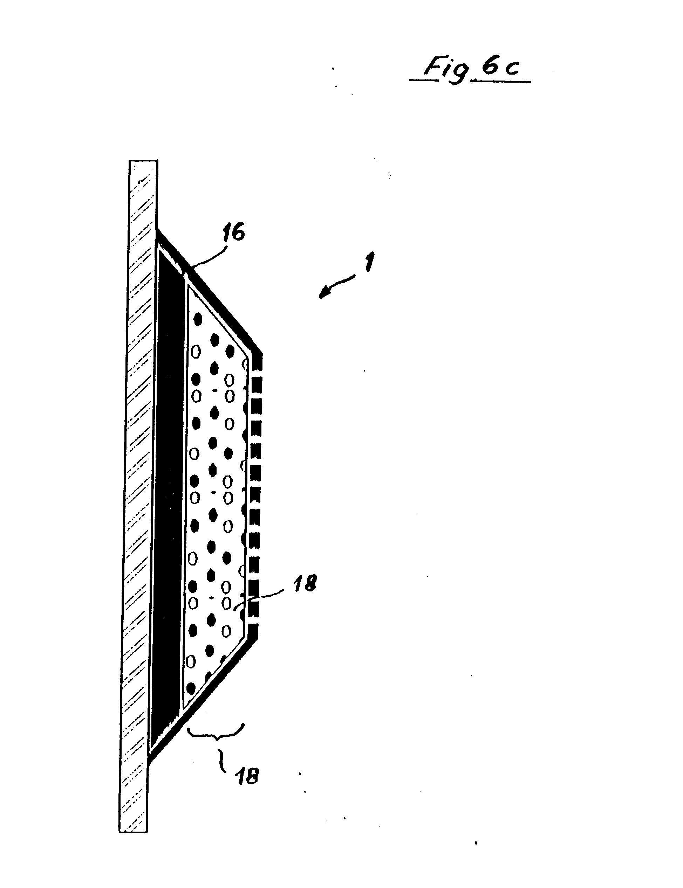

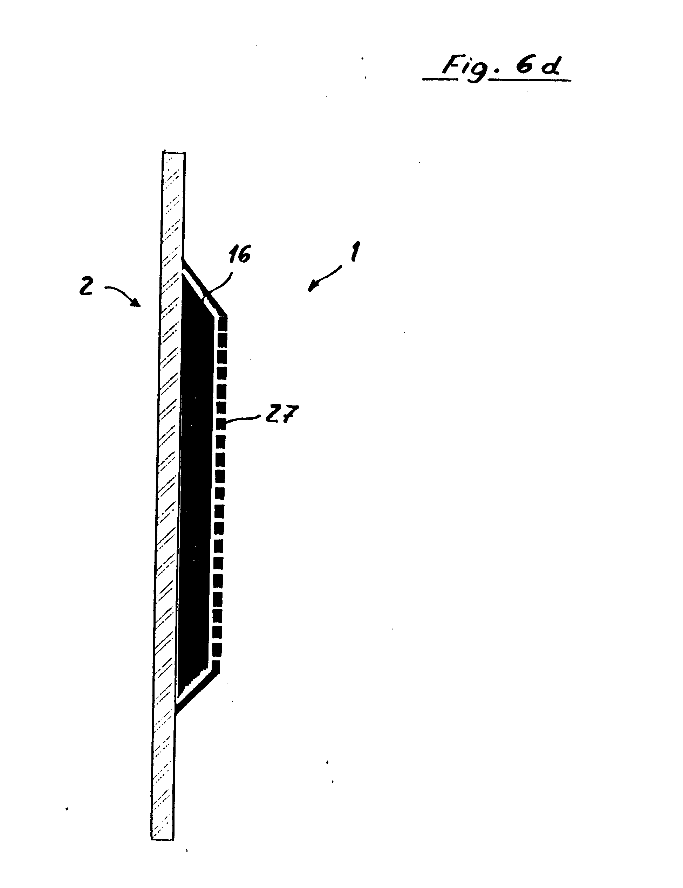

Fig. 6a zeigt einen erfindungsgemäßen Aufbau der eine doppelschichtige Membran oder Folie (13) beinhaltet, die zunächst im perforierten Abschnitt wasserdurchlässig ist und aus unterschiedlichen reaktiven Materialien besteht. Bei Feuchtigkeitsaufnahme bildet sich zwischen der doppelschichtigen Membran oder Folie (13) eine Sperrschicht (25), so daß nach ca. 2 Minuten die Durchlässigkeit nicht mehr gegeben ist.

Fig. 6b zeigt einen Aufbau der erfindungsgemäßen Vorrichtung (1), bei der sich an der Urinexponierten Seite eine Membran (26) befindet, die mit einer wasserlöslichen Schicht (24) überzogen ist. In dem Flüssigkeits- und/oder Milieusteuerungsbereich (18) ist eine Substanz (20) angeordnet die bei Feuchtigkeitsaufnahme mit der Membran (26) zeitverzögert reagiert und diese entsprechend abdichtet.

Fig. 6c zeigt ebenfalls eine erfindungsgemäße Vorrichtung (1), in deren Flüssigkeits- und/oder Milieusteuerungsbereich (18) Substanzen angeordnet sind, die durch Polymerisation oder Quellreaktionen den Indikator (16) wasserdicht einschließen.

Hierbei kommen chemische Reaktionen zum Einsatz, die mit Wasser physiologisch unbedenklich feste Substanzen bilden.

Dabei können auch Quellreaktionen zum Einsatz gelangen, die bei Wasseraufnahme einen dichten Film bilden können, wobei zu beachten ist, daß der Indikator (16) nach einer Zeit von ca. 2 Minuten hermetisch abgeschlossen wird.

Fig. 6d zeigt ein weiteres Ausführungsbeispiel der erfindungsgemäßen Vorrichtung (1), bei der diese aus der Trägerkarte (2) dem Indikator (16) und einem sich an der urinexponierten Seite direkt angrenzende Membran (27) besteht.

Die Membran (27) kann dabei sowohl semipermeabel als auch perforiert ausgestaltet sein und weist eine Aufrauhung auf.

Der Indikatorfarbstoff ist dabei unmittelbar mit Reagenzien oder Fixiersubstanzen vermischt und unmittelbar auf die Membran aufgebracht, wobei die Reagenzien und/oder Fixiersubstanzen bei Feuchtigkeitsaufnahme freigesetzt werden und die Indikatorreaktionen bzw. Indikatorverfärbungen stoppen.

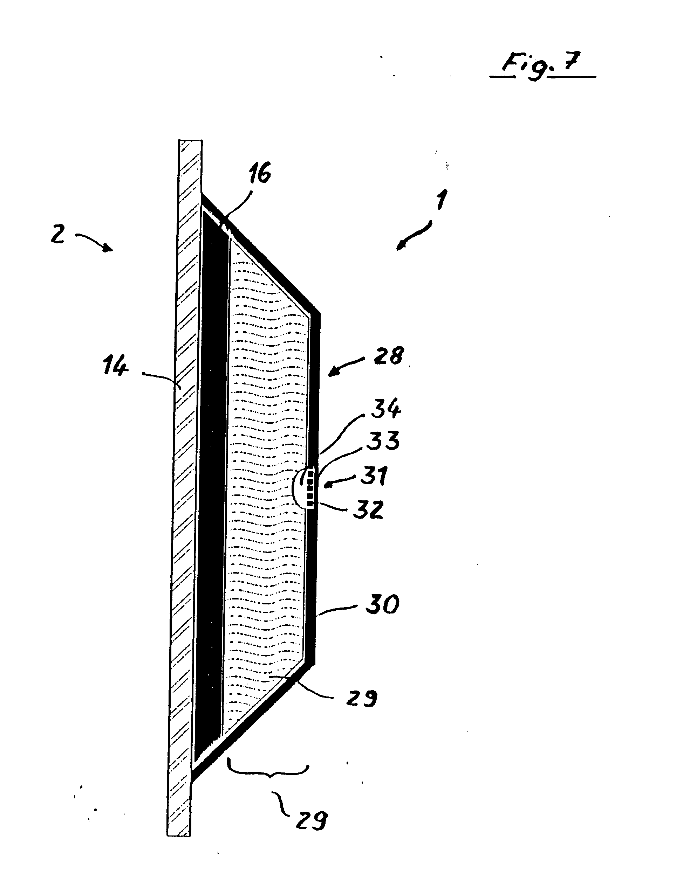

Gemäß einem neunten Ausführungsbeispiel, dargestellt in Fig. 7, arbeitet die erfindungsgemäße Vorrichtung (1) nach dem Vakutainer-Prinzip, wobei auch hier die erfindungsgemäße Vorrichtung einen sogenannten Träger, im hier vorliegenden Ausführungsbeispiel, die Check-up-Karte (2), aufweist und auf der urinexponierten Seite eine sogenannte Kapselfolie (28), die im hier vorliegenden Ausführungsbeispiel metallisiert ist.

Die Kapselfolie (28) bildet gemeinsam mit dem die Rückwand (14) bildenden Teil der Karte (2) auch im hiervorliegenden Ausführungsbeispiel eine trapezförmige Ausgestaltung und umschließt somit das Innere der erfindungsgemäßen Vorrichtung (1).

Im Inneren, unmittelbar an die Rückwand (14) angrenzend, ist der Indikator (16) angeordnet, wobei zwischem dem Indikator (16) bzw. der der Kapselfolie (28) zugewandten Seite des Indikators (16) und der Innenseite der Kapselfolie (28), auch hier ein sogenannter Flüssigkeits- und/oder Milieusteuerungsbereich bzw. Einrichtung (29) angeordnet ist. Im Inneren der erfindungsgemäßen Vorrichtung (1) besteht vor dem Einsatz ein Unterdruck, der erst während des Einsatzes aufgehoben wird.

Wie aus Fig. 7 ersichtlich, weist die urinexponierte Seite, das heißt die parallel zur Rückwand (14) verlaufende Seite (30) der Kapselfolie (28) etwa mittig angeordnet, eine Durchlaßöffnung (31) auf, die mit einer Perforation (32) versehen ist. Die Durchlaßöffnung (31) ist an der urinexponierten Seite mit einer wasserlöslichen Substanz (33) verschlossen.

Auf der der urinexponierten Seite abgewandten Seite der Kapselfolie (28) weist die Flüssigkeits- und/oder Milieusteuerungseinrichtung (29) im Bereich der Durchlaßöffnung (31) bzw. an die Durchlaßöffnung (31) angrenzend eine Einbuchtung (34) auf, die bei Aufnahme des Urins bzw. des wässrigen Milieus und nach Ausgleich des Unterdrucks zu einer Unterbrechung der Kapillarwirkung führt.

Der in der erfindungsgemäßen Vorrichtung (1) vorhandene Unterdruck entspricht gemäß dem Vakutainer-Prinzip der zu seperatisierenden Meßmenge.

Die Kapselfolie (28) ist im hier vorliegenden Ausführungsbeispiel mit Hilfe einer Metallbedampfung diffusionsdicht ausgestaltet, so daß der Unterdruck in der erfindungsgemäßen Vorrichtung (1) auch über eine längere Zeit gehalten werden kann.

Im Einsatz kommt es zu einer Auflösung der die Durchlaßöffnung (31) verschließenden wasserlöslichen Substanz (33), wobei diese auch aus einer chemisch reaktiven Substanz bestehen kann, so daß nach Auflösung dieser Substanz der Unterdruck in der Vorrichtung (1) dafür sorgt, daß anstehende Flüssigkeit in das Innere der Vorrichtung gesaugt wird, die dann zu den entsprechenden Messungen herangezogen wird.

Dabei steht die eintretende Flüssigkeitsmenge in einem vorbestimmten Verhältnis zu dem zuvor vorbestimmten Unterdruck.

Die hinter der Durchlaßöffnung (31) befindliche Einbuchtung (34) sorgt anschließend dafür, daß es nicht zu einer Kapillarwirkung bzw. zu einer Unterbrechung der Kapillarwirkung kommt und somit zu einer ungewollten Aufnahme weiterer Flüssigkeitsmengen.

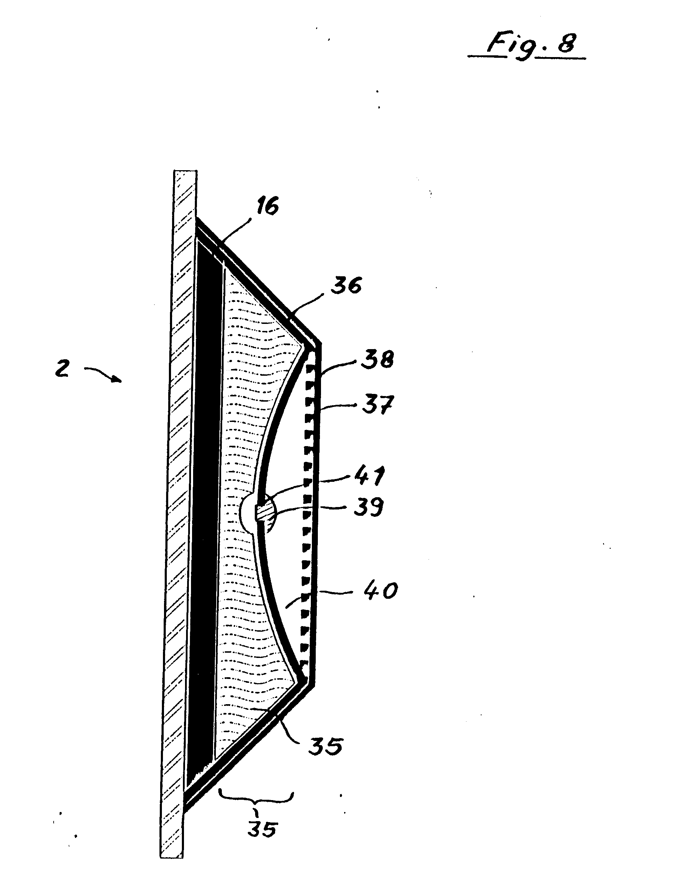

Gemäß dem Ausführungsbeispiel 9, dargestellt in Fig. 8, besteht die erfindungsgemäße Vorrichtung aus einem sie tragenden Träger (2), hier der bereits bekannten Check-up-Karte (2), einem Indikator (16), einer Flüssigkeits- und/oder Milieusteuerungseinrichtung (35), sowie einer elastischen Membran (36). Desweiteren weist die erfindungsgemäße Vorrichtung (1), gemäß Ausführungsbeispiel Fig. 8, eine perforierte Folie (37) auf, sowie einen sowohl die flexible Membran (36) als auch die perforierte Folie (37) umgebenden wasserlöslichen Schutzfilm (38).

Die Membran (36) weist in Blickrichtung Fig. 8 etwa mittig an ihrer unter Spannung gehaltenen Seite einen ebenfalls wasserlöslichen Verschluß (39) auf, wobei zwischen der perforierten Folie (37) und dem unter Spannung gehaltenen Teil der flexiblen Membran (36) ein Luftpolster (40) angeordnet ist.

Im Inneren der erfindungsgemäßen Vorrichtung (1) befindet sich ebenfalls, wie im vorangegangenen Ausführungsbeispiel, ein Unterdruck, auf den allerdings in einem weiteren Ausführungsbeispiel auch dann verzichtet werden kann, wenn das Luftpolster (40) einen Überdruck aufweist, so daß der ohne Vorspannung parallel zum Träger verlaufende Teil (36) der flexiblen Membran (36) durch diesen Überdruck angeordnet zwischen der perforierten Folie (37) und dem unter Vorspannung gehaltenen Teil der Membran (36) die Vorspannung dieses Teils verursacht.

Bei Benetzen der erfindungsgemäßen Vorrichtung mit einem wässrigen Milieu wie z.B. Urin wird zunächst einmal der wasserlösliche Schutzfilm (38) aufgelöst, so daß die zwischen dem vorgespannten Teil (36) der elastischen Membran (36) und der perforierten Folie (37) angeordnete Luft (40) entweichen kann, so daß das wässrige Milieu bzw. der Urin durch die perforierte Folie (37) in direktem Kontakt mit der vorgespannten Membran (36) und damit auch mit dem wasserlöslichen Verschluß (39) gelangt.

Durch diesen Kontakt wird der wasserlösliche Verschluß (39) aufgelöst, so daß, hervorgerufen durch den im Flüssigkeits- und/oder Milieusteuerungsbereich (29) herrschenden Unterdruck, das wässrige Milieu bzw. der Urin durch die Einlaßöffnung (41) in das Innere der erfindungsgemäßen Vorrichtung (1) gesaugt wird und über den Flüssigkeits- und/oder Milieusteuerungsbereich (29) an den Indikator (16) gelangen kann.

Dabei bestimmt sich die eindringende Flüssigkeitsmenge jedoch nicht nur durch den zuvor herrschenden Unterdruck in der erfindungsgemäßen Vorrichtung, sondern auch durch den durch die nun erfolgende Volumenvergrößerung des Innenraums und den damit entstehenden weiteren Unterdruck, da die vorgespannte Membran (36) nach Auflösen des wasserlöslichen Verschlusses (39) gegen die perforierte Folie (37) schnellt und so eine Volumenvergrößerung des Einrichtungsinneren verursacht.

Für den Fall, daß gemäß der Fig. 8 die Vorspannung der elastischen Membran (38) allein durch das Luftpolster (40) verursacht wurde, entsteht nach Auflösung des wasserlöslichen Schutzfilms (38) und des wasserlöslichen Verschlusses (39) ebenfalls ein Unterdruck durch Entspannen der Membran (36) und der damit einhergehenden Volumenvergrößerung des Innenraumes dervorrichtung (1), derjedoch geringer als der zuvor beschriebene Unterdruck ist, so daß eine nur geringere Flüssigkeitsmenge in das Innere der erfindungsgemäßen Einrichtung gelangt.

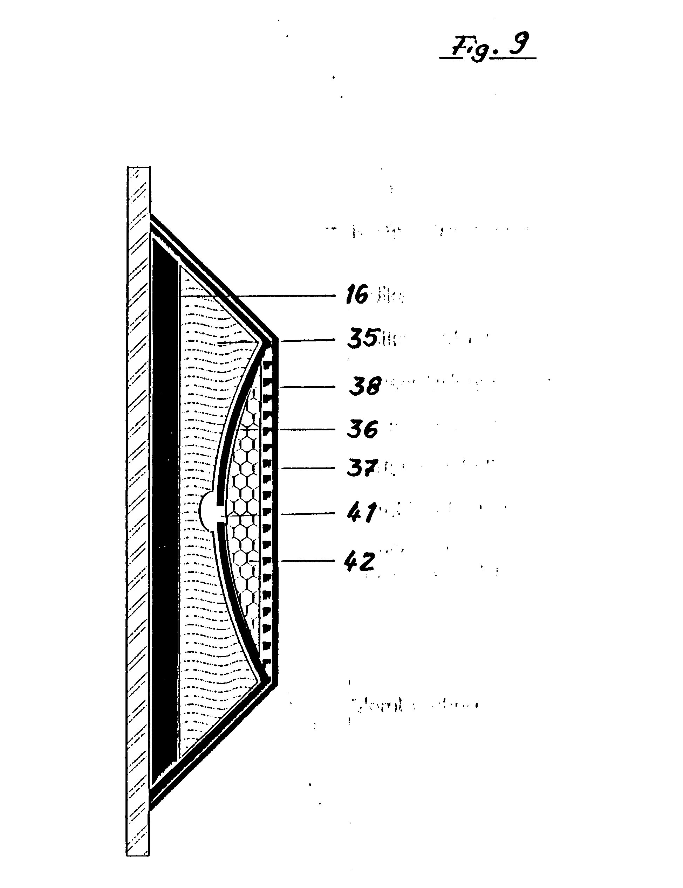

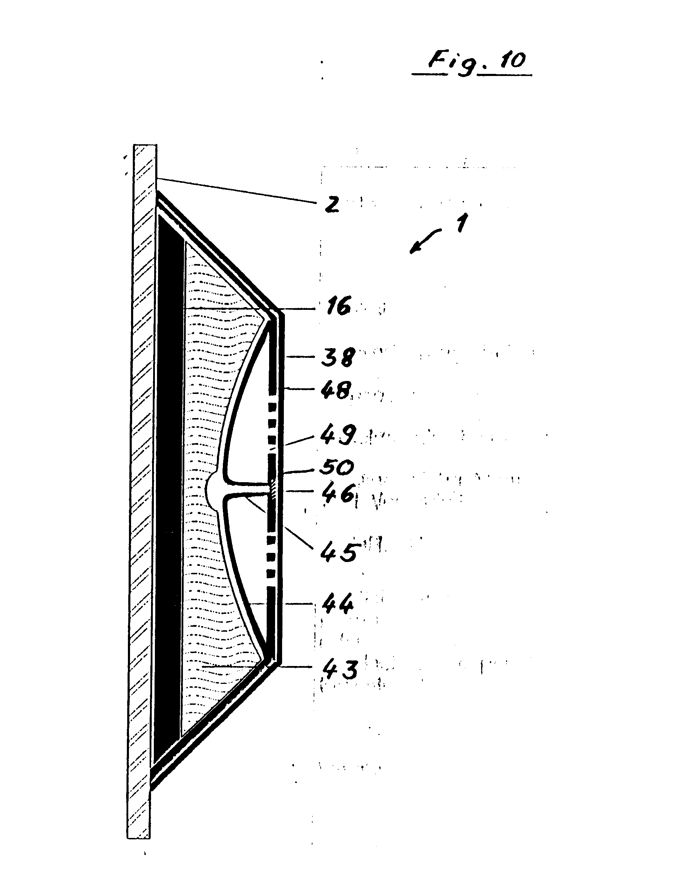

Das Ausführungsbeispiel gemäß Fig. 9 entspricht weitgehend dem Ausführungsbeispiel Fig. 8, wobei jedoch die Vorspannung der elastischen Membran (36) hier nicht durch einen Unterdruck oder durch ein zwischen der Membran (36) bzw. dem vorzuspannenden Teil der Membran (36) und der perforierten Folie (37) angeordnetes Luftkissen (40) erzeugt wird, sondern vielmehr durch eine zwischen der elastischen Membran (36) bzw. dem vorzuspannenden Abschnitt (36) der elastischen Membran (36) und der perforierten Folie (37) angeordneten wasserlöslichen Stütz- bzw. Fixiersubstanz (42), die die vorgespannte elastische Membran (36) in der aktiven Position fixiert.

Zweckmäßigerweise sollte diese Substanz (42) durch Hohlräume -Schaumstruktur - eine große Oberfläche haben, so daß nur eine relativ geringe Substanzmenge gelöst werden muß, die das Stützvolumen in Gegenwart von Feuchtigkeit sofort zusammenbrechen läßt.

Dabei kann sich die Substanz (42) bis in die Perforation der Folie (37) erstrecken oder sogar über diese hinaus gleichzeitig auch den Schutzfilm (38) bilden, das heißt einstückig mit diesem ausgestaltet sein.

Bei einem Zusammenbruch der Stützsubstanz (42) wird die elastische Membran (36) entspannt und saugt eine bestimmte Menge Flüssigkeit in das Vorrichtungsinnere.

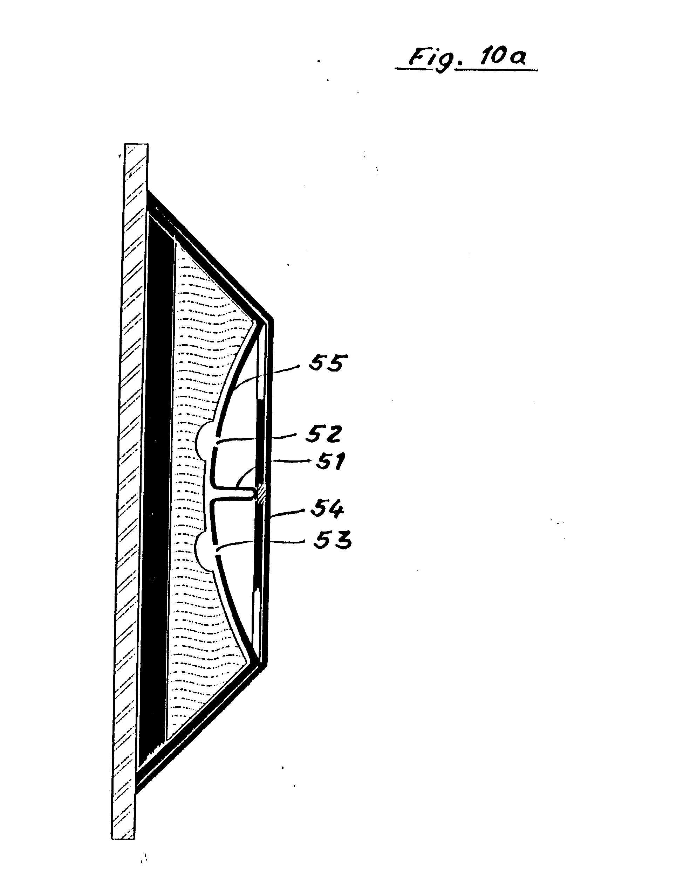

Die erfindungsgemäße Vorrichtung (1) gemäß Ausführungsbeispiel 9, dargestellt in Fig. 10, ist ebenfalls auf einen Träger, das heißt einer transparenten Karte (2) aufgebracht, wobei sich in unmittelbarer Nähe oder aber in direktem Kontakt zur Karteninnenseite im Bereich der Vorrichtung (1), das heißt an die im Inneren der erfindungsgemäßen Vorrichtung (1) befindliche Seite der Karte (2) der die Farbveränderungen anzeigende Indikator (16) anschließt.

Der Indikator (16), sowie die Flüssigkeits- und/oder Milieusteuerungseinrichtung (43), wird von einer elastischen Membran (36) umgeben, die gemeinsam mit der Karte (2) eine trapezförmige Ausgestaltung aufweist.

Der parallel zur Karte verlaufende Teil (44), das heißt der Blickrichtung Fig. 10, nach einer Entspannung senkrecht verlaufende Teil der Membran (36) ist hier elastisch vorgespannt, wobei er etwa mittig eine Einlaßkanüle (45) aufweist, die vor Einsatz der erfindungsgemäßen Vorrichtung (1) gegen eine wasserlösliche Membranfixierung (46) stößt, die gleichzeitig als Verschluß dient.

Desweiteren wird der durch die Vorspannung der Membran (36) entstehende teilkreisförmige Hohlraum (47) durch eine Kapselfolie (48) zur urinexponierten Seite hin abgeschlossen, die sogenannte Druckausgleichsöffnungen (49) aufweist.

Zusätzlich ist der gesamte Bereich der Kapselfolie (48) wie auch der Membran (36) an seiner urinexponierten Seite mit einem wasserlöslichen Schutzfilm (38) umgeben, so daß sichergestellt ist, daß das Innere der Vorrichtung (1) vor etwaigen Verschmutzungen geschützt ist.

Bei Einsatz der erfindungsgemäßen Vorrichtung (1) nach Ausführungsbeispiel 9 wird zunächst einmal durch Kontaktierung des wasserlöslichen Schutzfilmes (38) mit einem wässrigen Milieu bzw. Urin dieser aufgelöst, so daß hernach des wässrige Milieu bzw. der Urin an der ebenfalls wasserlöslichen Membranfixierung (46) ansteht, diese auflöst und es dadurch zu einer Entspannung der elastisch vorgespannten Membran (44) kommt und diese sich an die Kapselfolie (48) anlegt und dadurch die Druckausgleichsöffnungen (49) verschließt.

Dabei kann die, im Hohlraum zwischen der elastisch vorgespannten Membran (44) und der Kapselfolie (48) befindliche Luft durch die Druckausgleichsöffnungen (49) entweichen, so daß sich das Innere bzw. der Volumeninhalt der erfindungsgemäßen Vorrichtung (1) vergrößert und dadurch ein gewisser Unterdruck entsteht und Urin bzw. wässriges Milieu durch die Einlaßkanüle (45) in das Innere der Vorrichtung (1) gelangt.

Nach Ausgleich dieses Unterdrucks tritt dann kein weiteres wässriges Milieu bzw. Urin durch die Einlaßkanüle (45) in das Innere der Vorrichtung (1), insbesondere auch deswegen, weil die eine kapilare Wirkung aufweisende Flüssigkeits- und/oder Milieusteuerungseinrichtung (43) im Bereich der Einlaßkanüle (45) eine Einbuchtung (34) aufweist, die eine unmittelbare Kapilarwirkung verhindert.

Desweiteren wäre es ebenso denkbar, statt die Membran (36) elastisch vorzuspannen, die Flüssigkeits-und/oder Milieusteuerungseinrichtung (43) selbst ebenfalls entsprechend vorzuspannen, die dann nach dem Lösungsprozeß der Membranfixierung die Einlaßkanüle (45) dann durch die Verschlußöffnung (50) drückt, wodurch ebenfalls ein Unterdruck innerhalb der erfindungsgemäßen Einrichtung aufgebaut wird. Auch für diesen Fall ist die Flüssigkeits- und/oder Milieusteuerungseinrichtung (43), im Bereich des Flüssigkeitseinlasses mit einer halbkugelförmige Vertiefung (34) ausgestattet, die eine Kapillaritätsunterbrechung bewirkt bzw. sicherstellt.

Fig. 10a entspricht weitgehend dem in Fig. 10 dargestellten Prinzip, wobei hier jedoch keine Einlaßkanüle (45) vorgesehen ist, sondern vielmehr ein Stützelement (51), das genau wie die Einlaßkanüle (45) gehalten wird.

Die Flüssigkeitsmenge tritt hierdurch zwei Durchlaßöffnungen (52,53) ein, wobei die Folie (54) keine Perforation aufweist, sondern vielmehr vier über den Umfang gleichmäßig verteilte kreisrunde Durchlässe, die dann nach anliegen der elastischen Membran (55) geschlossen sind.

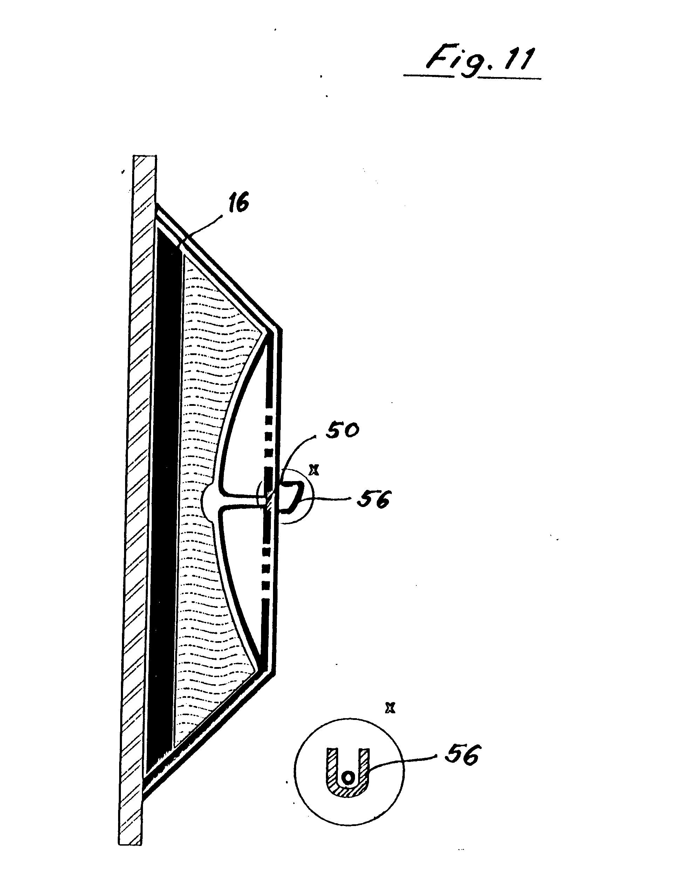

Das Ausführungsbeispiel gemäß Fig. 11 entspricht weitgehend dem Ausführungsbeispiel gemäß Fig. 10 und wird dadurch ergänzt, daß eine Auffangeinrichtung (56) für die Meßmenge vorgesehen ist.

Sollte in bestimmten Einsatzbereichen die kontinuierliche Benetzung der Karte bzw. des Indikators (16) schwierig sein, bietet sich eine derartige Einrichtung (56) an, die nach dem Lösen der Verschlußöffnung (50) die erforderliche Meßmenge vorhält.

Der Durchlaß der dosierten Meßmenge kann grundsätzlich auch an anderen Stellen erfolgen.

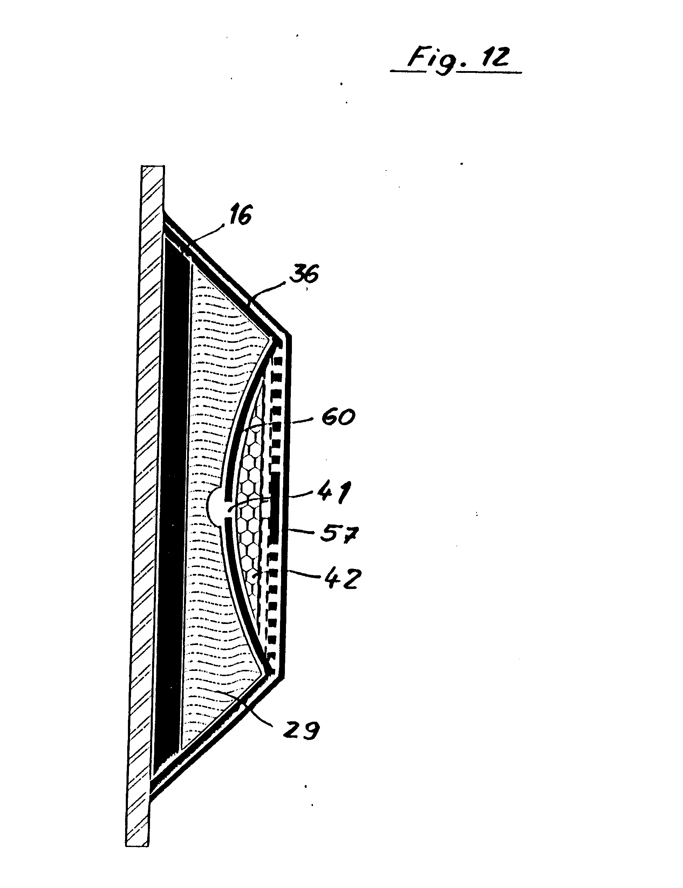

Das Ausführungsbeispiel 11 gemäß Fig. 12 zeichnet sich dadurch aus, daß eine vorgespannte elastische Membran (36,60) durch eine wasserlösliche Stütz- bzw. Fixiersubstanz (42) in der aktiven Position fixiert wird, wobei diese Fixier- bzw. Stützsubstanz (42) zweckmäßigerweise durch Hohlräume - Schaumstruktur - eine große Oberfläche haben sollte, damit lediglich eine relativ geringe Substanzmenge durch das wässrige Medium bzw. den Urin gelöst werden muß und das Stützvolumen in Gegenwart eines derartigen Mediums sofort zusammenbrechen läßt.

Eine weitere Möglichkeit bestünde darin, die Stütz- bzw. Fixiersubstanz (42) mit kleinen Kanälen, das heißt eine kapillare Wirkung aufweisenden Kunststoff zu versetzen, so daß bei Kontaktierung dieser Stützsubstanz (42) dann der Kunststoff das wässrige Medium, wie z.B. Urin, ansaugt und es zu einer Volumenerweiterung des Kunststoffes kommt, so daß die Stützsubstanz (42) hervorgerufen durch die Volumenerweiterung der Kapillare bzw. des Kunststoffes aufgebrochen und zerlegt wird. Dadurch ist entweder keine weitere Auflösung der Stützsubstanz (42) notwendig, oder aber es kann zu einer schnelleren Auflösung der Stützsubstanz (42) kommen.

Durch Auflösung bzw. Volumenzusammenbruch der Stütz- bzw. Fixiersubstanz (42) wird die elastische Membran (36) bzw. der vorgespannte Teil (60) entspannt und saugt eine bestimmte Menge Flüssigkeit, hervorgerufen durch einen dadurch entstehenden Unterdruck, in den Indikatorbereich.

Dabei entspricht dieses Lösungsbeispiel weitgehend dem Lösungsbeispiel 8, jedoch mit der Besonderheit, daß es durch die Entspannung der elastischen Membran (38) zu einem Abdichtungsprozeß kommt, in dem sich die elastische Membran (38) mit ihrer Durchlaßöffnung (41) gegen eine Dichtungsfläche (57) der perforierten Folie (37) legt, so daß keine weitere Flüssigkeit nachfließen kann.

Dieser Vorgang wird noch dadurch verstärkt, daß die entspannende Membran (38) gegen die Dichtungsfläche (52) mit Hilfe der ebenfalls vorgespannten Flüssigkeits- und/oder Milieusteuerungseinrichtung (29) oder aber der sich aufquellenden Flüssigkeits- und/oder Milieusteuerungseinrichtung (29) gedrückt wird.

In einem weiteren Ausführungsbeispiel ist der hier in Fig. 12 teilkreisförmig ausgestaltete Membranteil (38) derart vorgespannt, daß bei einem Quellvorgang der Flüssigkeits- und/oder Milieusteuerungseinrichtung (29) der teilkreisförmig ausgestaltete Membranteil übereinen Knickpunkt gedrückt wird und so in eine zweite gegen die Dichtungsfläche (57) anliegende stabile Position schnellt, um dann in dieser Position zu verharren und so den Innenraum abzudichten.

Eine Farbkonservierung kann dadurch vorgenommen werden, daß nach Beendigung der Indikatorreaktion und nicht mehr erfolgendem Nachfluß der Flüssigkeit das Indikatorträgermaterial Feuchtigkeit in einer bestimmten Zeit bindet, das heißt, den Meßbereich austrocknet, so daß keine weiteren Reaktionen im Indikator (16) mehr erfolgen können.

Voraussetzung hierfür ist, daß lediglich eine bestimmte Menge an Körperflüssigkeit in den Indikatorträger bzw. an den Indikator (16), wie hier durch die verschiedenen Ausführungsbeispiele sichergestellt, gelangen kann, die dann zeitverzögert gebunden wird.

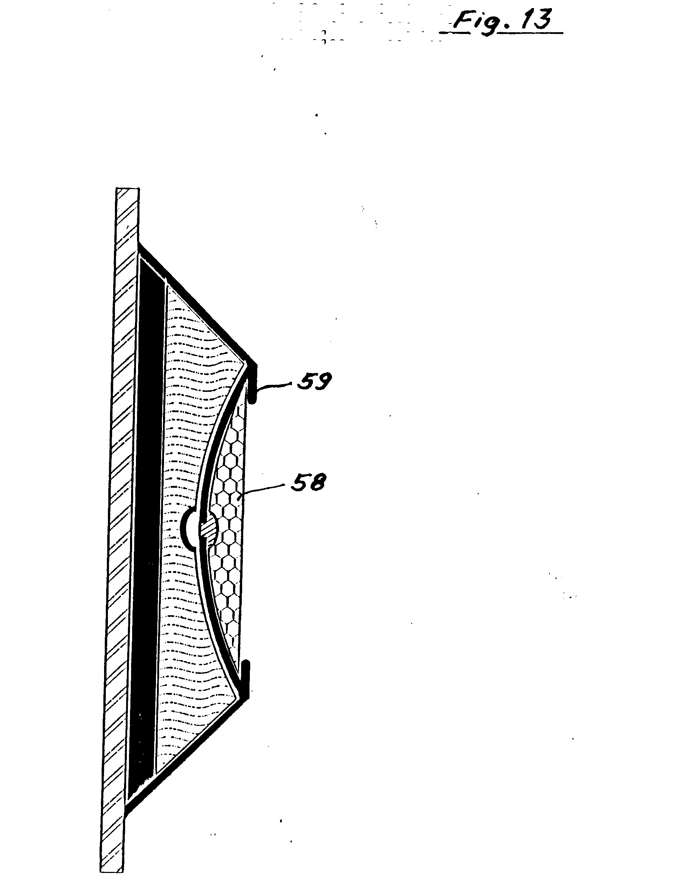

Bei einem weiteren Ausführungsbeispiel gemäß Fig. 13, wird die Fixier- und Stützsubstanz (58) durch einen als Kragen umlaufend ausgestalteten Vorsprung (59) gehalten, so daß das zu kontrollierende Milieu direkt an der Substanz (58) ansteht, während die übrigen Merkmale, wie bereits beschrieben, zum Einsatz gelangen können.

## Patentansprüche

1. Vorrichtung zur Erfassung und/oder Messung oder Kontrolle der Beschaffenheit, insbesondere chemischer Verhältnisse mit Hilfe von wenigstens einem Indikator in flüssigem Milieu, insbesonder wässrigen Milieu, wie z.B. Urin, mit einem die Vorrichtung aufnehmenden und/oder der Handhabung dienenden Träger und einer den Innenraum der vorrichtung wenigstens teilweise definierenden und umhüllenden Wandung dadurch gekennzeichnet, daß die Wandung (13;26;27;28; 36) wenigstens eine das flüssige Milieu über einen vorbestimmten zu definierenden Zeitraum in das Vorrichtungsinnere einlassenden Durchlaß (15;26;27;31;41;45) aufweist, daß eine das flüssige Milieu in eine Bewegung in das Vorrichtungsinnere veranlassende erste Einrichtung vorgesehen ist und eine den in das Vorrichtungsinnere stattfindenden Flüssigkeitseinlauf stoppende zweite Einrichtung vorgesehen ist.

2. Vorrichtung nach Anspruch 1, dadurch gekennzeichnet, daß der die Vorrichtung (1) aufnehmende Träger (2) im Bereich der Vorrichtung (1) eine den Vorrichtungsinnenraum begrenzende Wand (14) bildet und gemeinsam mit der den Vorrichtungsinnenraum wenigstens teilweise umhüllenden Wandung (13;26;27;28;36) die Innenraumgröße definiert.

3. Vorrichtung nach Anspruch 1 oder 2, dadurch gekennzeichnet, daß die Wandung (13;26;27;28;36) wenigstens teilweise diffusionsdicht ausgestaltet ist.

4. Vorrichtung nach einem der Ansprüche 1 bis 3, dadurch gekennzeichnet, daß die Wandung (13;26;27;28;36) an ihrer Außenseite wenigstens teilweise mit einem im flüssigen Milieu, insbesondere wässrigen Milieu, wie z.B. Urin, sich auflösenden Film (24;38), insbesondere durch Überzug abgedichtet ausgestaltet ist.

5. Vorrichtung nach einem der Ansprüche 1 bis 4, dadurch gekennzeichnet, daß die den Vorrichtungsinnenraum wenigstens teilweise umhüllende Wandung eine Membran (13;26;27;36,55) ist und der Durchlaß als eine in der Membran (13;26;27;55) angeordnete Perforation (15) ausgestaltet ist.

6. Vorrichtung nach einem der Ansprüche 1 bis 5, dadurch gekennzeichnet, daß die Membran aus einer ersten dem Innenraum zugewandten, einer durch das Milieu entstehenden, den Innenraum abdichtenden Grenzschicht (25) und einer zweiten dem Innenraum abgewandten Schicht besteht.

7. Vorrichtung nach einem der Ansprüche 1 bis 6, dadurch gekennzeichnet, daß zwischen dem den Durchlaß beinhaltenden Teil der Membran (15;30,44) und dem Indikator (16) eine Flüssigkeits- und/oder Milieusteuerungseinrichtung (18;23;29;35;43) vorgesehen ist.

8. Vorrichtung nach einem der Ansprüche 1 bis 7, dadurch gekennzeichnet, daß die Flüssigkeits- und/oder Milieusteuerungseinrichtung (18) eine mit Mikrokapseln und mit Kapillaren versehene die Einrichtung zur Veranlassung der Bewegung des flüssigen Milieus in das Vorrichtungsinnere darstellende Quellsubstanz (17) ist, daß die Kapselschale (20) semipermeabel wasserdurchlässig ausgestaltet ist und daß die zweite, den Flüssigkeitseinlauf stoppende Einrichtung ebenfalls die mikroverkapselte eine semipermeable Kapselschale (20) aufweisende und durch Aufnahme von Flüssigkeit ihre Volumengröße ändernde Quellsubstanz (17) ist.

9. Vorrichtung nach einem der Ansprüche 1 bis 7, dadurch gekennzeichnet, daß die Flüssigkeits- oder Milieusteuerungseinrichtung eine unverkapselte und mit Kapillaren versehene die Einrichtung zur Veranlassung der Bewegung des flüssigen Milieus in das Vorrichtungsinnere darstellende Quellsubstanz (17) ist und daß die zweite, den Flüssigkeitseinlauf stoppende Einrichtung ebenfalls die durch Aufnahme von Flüssigkeit ihre Volumengröße ändernde Quellsubstanz (17) ist.

10. Vorrichtung nach Anspruch 7, dadurch gekennzeichnet, daß zwischen der Flüssigkeits- und/oder Milieusteuerungseinrichtung (18;29;43) und dem Indikator (16) eine Durchlässe zu den Kapillaren aufweisende Sperrschicht (22) angeordnet ist.

11. Vorrichtung nach einem der Ansprüche 1 bis 7, dadurch gekennzeichnet, daß die Flüssigkeits und/oder Milieusteuerungseinrichtung (23) aus einem mit einem wasserlöslichen Hüllmaterial mikroverkapselten und mit Kapillaren versehenen die Einrichtung zur Veranlassung der Bewegung des flüssigen Milieus in das Vorrichtungsinnere darstellenden wasserundurchlässigen Dichtungsmittel (21) besteht und daß die zweite, den Flüssigkeitseinlauf stoppende Einrichtung ebenfalls die nach Auflösung der wasserlöslichen Kapselschale das Dichtungsmittel (21) freisetzende und die Kapillarität unterbrechende Flüssigkeits- und/oder Milieusteuerungseinrichtung (23) ist.

12. Vorrichtung nach einem der Ansprüche 1 bis 7, dadurch gekennzeichnet, daß die Flüssigkeits- und/oder Milieusteuerungseinrichtung (18) aus einem hydrophoben Material besteht, mit Kapillaren versehen ist und daß die Oberfläche der Kapillaren mit einer wasserlöslichen hydrophilen Substanz versehen sind.

13. Vorrichtung nach Anspruch 1 oder 2, dadurch gekennzeichnet, daß die den Vorrichtungsinnenraum wenigstens teilweise umhüllende Wandung eine Folie (28) ist.

14. Vorrichtung nach Anspruch 13, dadurch gekennzeichnet, daß der Durchlaß (31) als eine in der Folie (28) angeordnete Perforation (33) ausgestaltet ist.

15. Vorrichtung nach Anspruch 13 oder 14, dadurch gekennzeichnet, daß der Durchlaß (39) wenigstens an seiner Außenseite durch eine wasserlösliche oder chemisch/physikalisch reaktive Substanz (33) verschlossen ausgestaltet ist.

16. Vorrichtung nach Anspruch 1 oder 2 und 13 bis 15, dadurch gekennzeichnet, daß die Folie (28) eine diffusionsdichte metallbedampfte Folie (28) ist.

17. Vorrichtung nach Anspruch 1 oder und 13 bis 16, dadurch gekennzeichnet, daß das Vorrichtungsinnere, insbesondere im Bereich der die erste und zweite Einrichtung darstellenden Flüssigkeits- und/oder Milieusteuerungseinrichtung (29,43) einen, die einfließende Flüssigkeitsmenge wenigstens teilweise bestimmenden Unterdruck aufweist.

18. Vorrichtung nach einem der Ansprüche 1 bis 17, dadurch gekennzeichnet, daß im Bereich des Durchlasses (31;41; 45) die Flüssigkeits- und/oder Milieusteuerungseinrichtung (29;35;43) eine wenigstens teilkugelförmige Einbuchtung (34) aufweist.

19. Vorrichtung nach einem der Ansprüche 1 bis 18, dadurch gekennzeichnet, daßwenigstens der den Durchlaß (31;41; 45) aufweisende Teil der den Innenraum der Vorrichtung (1) wenigstens teilweise definierenden und umhüllenden Wandung (13,26;27;28;36) elastisch ausgestaltet ist.

20. Vorrichtung nach Anspruch 17 und/oder 19, dadurch gekennzeichnet, daß der elastisch ausgestaltete Teil (30;44) der Membran (36), hervorgerufen durch den im Vorrichtungsinneren herrschenden Unterdruck in eine vorgespannte nach innen gerichtete Position angeordnet ist.

21. Vorrichtung nach Anspruch 19 oder 20, dadurch gekennzeichnet, daß im Bereich des den Durchlaß (41;45) aufweisenden Teiles der Membran eine perforierte Folie (37,48) derart angeordnet ist, daß zwischen der unter Vorspannung ausgerichteten Membran (30,36,44) und der der vorgespannten Membran zugewandten Seite der perforierten Folie (37; 48) ein Hohlraum (47) ausgebildet ist.

22. Vorrichtung nach Anspruch 21, dadurch gekennzeichnet, daß die perforierte Folie (37) dem Durchlaß gegenüberliegend eine nach Anliegen des elastischen Membranteiles (60) an der Folie (37) den Durchlaß (41) verschließende Dichtungsfläche (57) aufweist.

23. Vorrichtung nach Anspruch 21 oder 22, dadurch gekennzeichnet, daß die Membran (38) mit der Dichtungfläche (57) verklebend ausgestaltet ist.

24. Vorrichtung nach Anspruch 21, dadurch gekennzeichnet, daß ein wenigstens die perforierte Folie abdichtender wasserlöslicher Schutzfilm (38) vorgesehen ist.

25. Vorrichtung nach Anspruch 19, 21 und 23, dadurch gekennzeichnet, daß der elastisch ausgestaltete Teil der Membran (60), hervorgerufen durch ein zwischen dem Membranteil (60) und der perforierten Folie (37) angeordnetes Luftpolster (40) in einer vorgespannten nach innen gerichteten Position angeordnet ist.

26. Vorrichtung nach Anspruch 19, 21 und 23, dadurch gekennzeichnet, daß zwischen dem elastisch ausgestalteten Teil der Membran (60) und der perforierten Folie (37) eine den elastisch ausgestalteten Teil der Membran (60) in nach innen gerichteter Vorspannung haltende Stütz- oder Fixiersubstanz (42) angeordnet ist.

27. Vorrichtung nach Anspruch 25, dadurch gekennzeichnet, daß die Stütz- oder Fixiersubstanz (42) milieulöslich oder chemisch reaktiv ausgestaltet ist.

28. Vorrichtung nach Anspruch 25 oder 26, dadurch gekennzeichnet, daß die Stütz- oder Fixiersubstanz (42) Hohlräume aufweist.

29. Vorrichtung nach Anspruch 25, dadurch gekennzeichnet, daß die Stütz- oder Fixiersubstanz (42) mit einer Kapillare aufweisenden und ihr Volumen bei Aufnahme von flüssigem Milieu vergrößernder Substanz, insbesondere Kunststoff versetzt ist.

30. Vorrichtung nach einem der Ansprüche 20 bis 28, dadurch gekennzeichnet, daß die Flüssigkeits- und/oder Milieusteuerungseinrichtung (18,35,43) bei Aufnahme des zu erfassenden oder messenden flüssigen Milieus volumenvergrößernd und die Membran (36) in Richtung Folie (37) drückend ausgestaltet ist.

31. Vorrichtung nach einem der Ansprüche 1 bis 28, dadurch gekennzeichnet, daß der Durchlaß mit einem nach außen gerichteten Einlaßkanal (45) versehen ist.

32. Vorrichtung nach einem der Ansprüche 19 bis 29, dadurch gekennzeichnet, daß die Folie (48) wenigstens eine Druckausgleichsöffnung (49) und eine zur Durchführung des Einlaßkanals (45) geeignete Öffnung (50) aufweist und daß die Folie (48) derart angeordnet ist, daß zwischen der perforierten Folie (48) und dem unter Vorspannung ausgerichteten elastischen Teil (44) der Membran (36) ein Hohlraum (47) ausgebildet ist.

33. Vorrichtung nach Anspruch 26 bis 27, dadurch gekennzeichnet, daß die zur Durchführung des Einlaßkanals (45) geeignete Öffnung (50) mittels eines wasserlöslichen oder chemisch/physikatisch reaktiven Verschlusses (46) verschlossen ist und daß der Einlaßkanal (45) den elastisch ausgestalteten Teil (44) der Membran (36) unter Vorspannung haltend in direkten Kontakt mit dem Verschluß (46) stehend angeordnet und ausgebildet ist.

34. Vorrichtung nach Anspruch 28, dadurch gekennzeichnet, daß die zur Durchführung des Kanals (45) geeignete Öffnung (50) mit einer Auffangeinrichtung (56) versehen ist.

35. Vorrichtung nach einem der Ansprüche 1 bis 34, dadurch gekennzeichnet, daßwenigstens die Innenwandung des Durchlasses hydrophil ausgestaltet ist.
